# EUROPEAN PATENT SPECIFICATION

(11) **EP 1 814 595 B1**
(45) Date of publication and mention of the grant of the patent: **08.01.2014**
(21) Application number: 05810600.6
(22) Date of filing: 22.11.2005
(51) Int. Cl.: A61K 48/00, A61P 35/00, A61K 39/395, A61K 31/7088

(54) **TREATMENT OF CANCER WITH A COMBINATION OF AN AGENT THAT PERTURBS THE EGF SIGNALING PATHWAY AND AN OLIGONUCLEOTIDE THAT REDUCES CLUSTERIN LEVELS**
BEHANDLUNG VON KREBS MIT EINER KOMBINATION AUS EINEM WIRKSTOFF, DER DEN EGF-SIGNALWEG STÖRT, UND EINEM OLIGONUKLEOTID, DAS DIE CLUSTERINMENGE REDUZIERT
TRAITEMENT DU CANCER AVEC COMBINAISON D'UN AGENT QUI PERTURBE LA VOIE DE SIGNALISATION EGF ET D'UN OLIGONUCLEOTIDE QUI REDUIT LES NIVEAUX DE CLUSTERINE

(30) Priority: 23.11.2004 US 522948 P; 24.11.2004 US 522960 P
(43) Date of publication of application: 08.08.2007
(73) Proprietor: THE UNIVERSITY OF BRITISH COLUMBIA, Vancouver, British Columbia V6T 1Z3 (CA)
(72) Inventor: GLEAVE, Martin, Vancouver, British Columbia V6R 1E8 (CA); ZUPI, Gabriella, I-00158 Roma (IT)
(74) Representative: Austin, Hedley William
(86) International application number: PCT/CA2005/001775
(87) International publication number: WO 2006/056054

(56) References cited:
- WO-A-00/49937
- WO-A2-00/49937
- US-A1- 2003 166 591
- US-A1- 2004 006 106
- US-A1- 2004 096 882
- BIROCCIO ANNAMARIA ET AL: "Antisense clusterin oligodeoxynucleotides increase the response of HER-2 gene amplified breast cancer cells to trastuzumab" JOURNAL OF CELLULAR PHYSIOLOGY, vol. 204, no. 2, August 2005 (2005-08), pages 463-469, XP002517624 ISSN: 0021-9541
- MILELLA MICHELE ET AL: "Trastuzumab down-regulates Bcl-2 expression and potentiates apoptosis induction by Bcl-2/Bcl-XL bispecific antisense oligonucleotides in HER-2 gene--amplified breast cancer cells." CLINICAL CANCER RESEARCH : AN OFFICIAL JOURNAL OF THE AMERICAN ASSOCIATION FOR CANCER RESEARCH 15 NOV 2004, vol. 10, no. 22, 15 November 2004 (2004-11-15), pages 7747-7756, XP002517625 ISSN: 1078-0432
- TROUGAKOS IOANNIS P ET AL: "Silencing expression of the Clusterin/Apolipoprotein J gene in human cancer cells using small interfering RNA induces spontaneous apoptosis, reduced growth ability, and cell sensitization to genotoxic and oxidative stress." CANCER RESEARCH, vol. 64, no. 5, 1 March 2004 (2004-03-01), pages 1834-1842, XP002517626 ISSN: 0008-5472
- MIYAKE ET AL: "SYNERGISTIC ANTITUMOR ACTIVITY BY COMBINED TREATMENT WITH GEMCITABINE AND ANTISENSE OLIGODEOXYNUCLEOTIDE TARGETING CLUSTERIN GENE IN AN INTRAVESICAL ADMINISTRATION MODEL AGAINST HUMAN BLADDER CANCER KOTCC-1 CELLS" JOURNAL OF UROLOGY, BALTIMORE, MD, US, vol. 171, no. 6, 1 June 2004 (2004-06-01), pages 2477-2481, XP005532561 ISSN: 0022-5347
- GLEAVE M ET AL: "CLUSTERIN AND IGFBPS AS ANTISENSE TARGETS IN PROSTATE CANCER" ANNALS OF THE NEW YORK ACADEMY OF SCIENCES, NEW YORK ACADEMY OF SCIENCES, NEW YORK, NY, US, 1 January 2003 (2003-01-01), pages 95-104, XP009039799 ISSN: 0077-8923
- JANSEN B ET AL: "Antisense therapy for cancer-the time of truth" LANCET ONCOLOGY, LANCET PUBLISHING GROUP, LONDON, GB, vol. 3, no. 11, 1 November 2002 (2002-11-01), pages 672-683, XP004810743 ISSN: 1470-2045
- FRIEDLANDER ET AL: "ErbB-directed immunotherapy: Antibodies in current practice and promising new agents" IMMUNOLOGY LETTERS, ELSEVIER BV, NL, vol. 116, no. 2, 26 December 2007 (2007-12-26), pages 126-140, XP022513146 ISSN: 0165-2478
- PEIPP M ET AL: "Effector mechanisms of therapeutic antibodies against ErbB receptors" CURRENT OPINION IN IMMUNOLOGY, ELSEVIER, OXFORD, GB, vol. 20, no. 4, 1 August 2008 (2008-08-01), pages 436-443, XP023612043 ISSN: 0952-7915 [retrieved on 2008-07-01]
- CHUNG J. ET AL: 'Enhanced chemosensitivity of bladder cancer cells to cisplatin by suppression of clusterin in vitro' CANCER LETT vol. 203, no. 2, January 2004, pages 155 - 161, XP008118434
- TROUGAKOS I.P. ET AL: 'Silencing expression of the clusterin/apolipoprotein J gene in human cancer cells using small interfering RNA induces spontaneous apoptosis, reduced growth ability, and cell sensitization to genotoxic and oxidative stress' CANCER RES. vol. 64, no. 5, 01 March 2004, pages 1834 - 1842, XP002517626
- BIROCCIO A. ET AL: 'The future of antisense therapy: combination with anticancer treatments' ONCOGENE vol. 22, 2003, pages 6579 - 6588, XP008118435
- CIARDIELLO F., TORTORA G.: 'A novel approach in the treatment of cancer: targeting the epidermal growth factor receptor' CLIN CANCER RES vol. 7, no. 10, October 2001, pages 2958 - 2970, XP001153753
- BIROCCIO A. ET AL: 'Antisense clusterin oligodeoxynucleotides increase the response of HER-2 gene amplified breast cancer cells to Trastuzumab' J CELL PHYSIOL vol. 204, no. 2, August 2005, pages 463 - 469, XP002517624

## Description

This application claims priority from US Provisional Applications 60/522,948 filed November 23, 2004 and 60/522,960 filed November 24, 2004.

### FIELD OF THE INVENTION

The present application relates to treatment of cancer in a mammalian subject using a combination of therapeutic agents, one of which is an oligonucleotide effective to reduce the amount of clusterin, also known as testosterone-repressed prostate message-2 (TRPM-2) in the cancer cells, and the other of which is an agent that perturbs the EGF cell signaling pathway, and also stimulates the expression of clusterin as a consequence of its action on the target. Examples of agents that perturb the EGF signaling pathway include agents that target HER-2.

### BACKGROUND OF THE INVENTION

After lung cancer, breast cancer is the.second leading cause of cancer deaths in women. According to the World Health Organization, more than 1.2 million people will be diagnosed with breast cancer this year worldwide, and The American Cancer Society estimates that in 2004, over 200,000 women in the United States will be diagnosed with invasive breast cancer (Stages I-IV), and about 40,000 women and almost 500 men will die from breast cancer in the United States in 2004.

The incidence rate of breast cancer (number of new breast cancers per 100,000 women) increased by approximately 4% during the 1980s but leveled off to 100.6 cases per 100,000 women in the 1990s.

Standard treatments include surgery, radiation, chemotherapy and hormonal therapies. Each of these treatments has drawbacks including loss of breast tissue, illness associated with radiation or chemotherapy, reproductive and hormonal side effects, and unreliable survival rates.

Thus breast cancer is a serious disease, fatal in many cases, and requires improved treatments to reduce fatalities and prevalence.

Clusterin or "TRPM-2" is a ubiquitous protein, with a diverse range of proposed activities. In prostate epithelial cells, expression of clusterin increases immediately following castration, reaching peak levels in rat prostate cells at 3 to 4 days post castration, coincident with the onset of massive cell death. These results have led some researchers to the conclusion that clusterin is a marker for cell death, and a promoter of apoptosis. On the other hand, Sertoli cells and some epithelial cells express high levels of clusterin without increased levels of cell death. Sensibar et al., (1995)[1] reported on *in vitro* experiments performed to more clearly elucidate the role of clusterin in prostatic cell death. The authors used LNCaP cells transfected with a gene encoding clusterin, and observed whether expression of this protein altered the effects of tumor necrosis factor α (TNFα), to which LNCaP cells are very sensitive. Treatment of the transfected LNCaP cells with TNFα resulted in a transient increase in clusterin levels for a few hours, but these levels had dropped by the time DNA fragmentation preceding cell death was observed.

United States published patent application US 20030166591 discloses the use of antisense therapy which reduces the expression of clusterin for the treatment of cancer of prostate and renal cell cancer.

US Patent No. 6,383,808 discloses compositions, particularly oligonucleotides, and methods for modulating the expression of clusterin.

United States published patent application 2004096882 discloses RNAi therapeutic probes targeting cancer associated proteins including clusterin.

United States published patent application US2004053874 discloses antisense modulation of clusterin expression.

United States published patent application US 2003166591 discloses cluserin antisense therapy using an oligonucleotide having 2'-O-(2-methoxy)ethyl modifications.

United States published patent application US 2003158130 discloses the use of chemotherapy-sensitization and radiation-sensitization of cancer by antisense clustgerin oligodeoxynucleotides.

### SUMMARY OF THE INVENTION

Applicants have found that agents that perturb the HER-2 signalling pathway and that are known to be useful in the treatment of cancer can result in increased expression of the protein clusterin. Since clusterin can provide protection against apoptosis, this secondary effect detracts from the efficacy of the therapeutic agent. In overcoming this, the present invention provides a combination of therapeutic agents that is useful in the treatment of cancer. The combination according to the invention is defined in claim 1.

The combination of the invention is useful in a method for treating cancer in a mammalian subject, comprising administering to the subject the known therapeutic agent and an oligonucleotide effective to reduce the amount of clusterin in the cancer cells.

The cancer may be breast cancer, osteosarcoma, lung cancer, pancreatic cancer, salivary gland cancer, colon cancer, prostate cancer, endometrial cancer, and bladder, for example.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

In drawings which illustrate embodiments of the invention,
Figure 1 A shows a cytofluorimetric analysis of HER-2 protein expression in BT474 cells untreated (gray area), and treated with 10 (thin line), 25 µg/ml (thick line) of trastuzumab for 48h, and negative control (dotted area);
Figure 1 B shows the number of adherent cells (black columns) and percentage of apoptotic cells (white columns) in BT474 cells, untreated and treated with 10 and 25 µg/ml of trastuzumab for 48h;
Figure 2 shows data for cells treated with 500nM of either clusterin ASO or mismatch (MM) control oligodeoxynucleotide for 6h, followed by exposure to 25 µg/ml of trastuzumab or control medium, 48h after treatment;
Figure 3 is a histogram showing the relative percentages of cells in the different phases of cell cycle (measured using propidium iodide staining and flow cytometry) after treatment with 500 nM of either clusterin ASO or MM control oligonucleotide for 6h followed by exposure to 25 µg/ml of trastuzumab or control medium; and
Figure 4 shows the cytofluorimetric analysis of annexin V/PI staining for cells that were treated with 500 nM of either clusterin ASO or MM control oligonucleotide for 6h, followed by exposure to 25 µg/ml of trastuzumab or control medium. Annexin V-positive cells are highlighted in the box.

### DETAILED DESCRIPTION OF THE INVENTION

### Definitions

As used herein, the term "HER-2 cell signalling pathway" refers to the intracellular pathway that is stimulated upon binding of a ligand to HER-2, a member of the epidermal growth factor receptor (EGFr) family. The EGFr family comprises EGFR, HER-2, HER-3 and HER-4. The EGFr family lies at the beginning of a complex signal transduction/communication pathway that modulates cell proliferation, survival, migration and differentiation.

As used herein, the phrase "an agent that perturbs the HER-2 cell signalling pathway" refers to any agent which is capable of disrupting the HER-2 cell signal, and includes a monoclonal antibody specific for HER-2, a small molecule inhibitor of normal binding to HER-2, an antisense oligonucleotide that specifically inhibits expression of HER-2, or a peptide capable of interfering with the signalling function of HER-2.

As used herein, the term "clusterin" refers to the glycoprotein originally derived from ram rete testes, and to homologous proteins derived from other mammalian species, including humans, whether denominated as clusterin or an alternative name. The sequences of numerous clusterin species are known. For example, the sequence of human clusterin is reported by Wong et al., (1994) [2], and in NCBI sequence accession number NM_001 831 and is set forth in Seq. ID No. 1. In this sequence, the coding sequence spans bases 48 to 1397.

As used herein, the term "amount of clusterin" refers to the amount of clusterin which is present in a form which is functional to provide anti-apoptotic protection. The effective amount of clusterin may be reduced through restricting production of clusterin (at the transcription or translation level) or by degrading clusterin at a rate faster than it is being produced. Further, it will be appreciated that inhibition occurs when the clusterin would otherwise be present if the antisense oligonucleotide had not been administered.

As used in the specification, "antisense oligonucleotide" refers to stretches of single-stranded DNA, usually chemically modified, whose sequence (3' → 5') is complementary to the sense sequence of a molecule of mRNA. Antisense molecules thereby effectively inhibit gene expression by forming RNA/DNA duplexes, and offer a more targeted option for cancer therapy than chemotherapy or radiation. Antisense is believed work by a variety of mechanisms, including physically blocking the ability of ribosomes to move along the messenger RNA, and hastening the rate at which the mRNA is degraded within the cytosol. The abbreviation ASO may also be used to refer to an antisense oligonucleotide.

As used in the specification and claims of this application, the term "combination" refers to an assemblage of reagents for use in therapy either by simultaneous or contemporaneous administration. Simultaneous administration refers to administration of an admixture (whether a true mixture, a suspension, an emulsion or other physical combination) of the agent that perturbs the HER-2 cell signaling pathway and the oligonucleotide. In this case, the combination may be the admixture or separate containers of the agent and the oligonucleotide that are combined just prior to administration. Contemporaneous administration refers to the separate administration of the agent and the oligonucleotide at the same time, or at times sufficiently close together that a synergistic activity relative to the activity of either the agent or the oligonucleotide alone is observed. In this, the combination comprises separate containers of the agent and the oligonucleotide

### Agents that Perturb the HER-2 signalling pathway

As noted above, the present invention makes use of an agent that perturbs the HER-2 cell signalling pathway. This agent can be any agent which is capable of disrupting the HER-2 cell signal, and includes an antibody specific for any of the members of the HER-2, a small molecule inhibitor of normal binding to HER-2, an antisense oligonucleotide that specifically inhibits expression of HER-2, or a peptide agent capable of interfering with the signalling function of HER-2.

The agent is one that perturbs the EGF signalling pathway by interaction with HER-2. HER-2, also known as ERBB2, or human epidermal growth factor receptor 2, helps control how cells grow, divide, and repair themselves. There has been extensive research done on the HER-2 gene and the role of the HER-2 protein in cancer dating from the 1980s. The HER-2 gene directs the production of special proteins, called HER-2 receptors. HER-2 is overexpressed in about a third of all breast cancers and is the target of trastuzumab.

One type of agent that can be used to interact with the HER-2 receptor and perturb the EGF signalling pathway is a pharmaceutical monoclonal antibody. The antibody may be specific for, and bind to, the HER-2 receptor. Alternately the antibody may bind to a related receptor and affect the HER-2 pathway in that way. This antibody may be trastuzumab, which is believed to block the HER-2 receptors when there is overexpression, and thereby block tumor growth and development. Trastuzumab, sold under the brand name Herceptin™, is a recombinant monoclonal antibody administered intravenously to treat breast cancer. Trastuzumab is currently used in combination with paclitaxel and is indicated for treatment of patients with metastatic breast cancer whose tumors over-express the HER-2 protein.

Other agents capable of perturbing the HER-2 cell signaling pathway include antisense agents capable of blocking *HER-2* expression. US Patent publication 2003105051 discloses nucleic acid therapeutics for conditions related to levels of HER-2, and US patent nos. 5,910,583 and 6,365,345 disclose antisense nucleic acids for the prevention and treatment of disorders in which expression of c-erbB or erbB2 play a role

The examples below demonstrate that increased clusterin expression is observed when trastuzumab is used to target HER-2 in breast cancer cells.

Small molecules capable of acting as the agent to perturb HER-2 cell signaling include, for example, those disclosed in published patent documents US 5, 721, 237, WO 03035843, and EP1131304. Peptides and peptide mimetics capable of acting as the agent to interact with HER-2 receptor and perturb the EGF signaling pathway include those such as adamanolol and disclosed in, for example, published patent documents CA2373721 and US2004006106.

As an alternative to agents that interact with HER-2, agents that interact with other members of the EGFr family may also be employed in the invention. For example, Erbitux™ (cetuximab) is a known pharmaceutical monoclonal antibody that targets the EGF cell signalling pathways via EGFr in colon and head and neck carcinoma. Oligonucleotides

### Antisense Oligonucleotides (ASO)

Antisense oligonucleotides are synthetic polymers made up of monomers of deoxynucleotides like those in DNA. In the present application, the term antisense oligonucleotides includes antisense oligodeoxynucleotides.

The antisense oligonucleotides for use in the combination of the invention for treatment of cancer in humans are complementary to the nucleotide sequence of human clusterin as set forth in Seq. ID No. 1. In specific embodiments, the antisense oligonucleotide may span either the translation initiation site or the termination site of clusterin. The antisense oligonucleotide comprises and may consist essentially of an oligonucleotide selected from the group consisting of Seq. ID. Nos.: 2 to 19, or more specifically Seq. ID. No. 4, Seq. ID. No. 5 and Seq. ID. No.12. As used in the specification and claims of this application, the phrase "consist essentially of" means that the oligonucleotide contains just the bases of the identified sequence or such bases and a small number of additional bases that do not materially alter the antisense function of the oligonucleotide In order to avoid digestion by DNAse, antisense oligonucleotides and oligonucleotides are often chemically modified. For example, phosphorothioate oligodeoxynucleotides are stabilized to resist nuclease digestion by substituting one of the non-bridging phosphoryl oxygen of DNA with a sulfur. Increased antisense oligonucleotide stability can also be achieved using molecules with 2-methoxyethyl (MOE) substituted backbones as described generally in US Patent No. 6, 451, 991, , and US published patent application US-2003-0158143-A1. Thus, in the combination of the invention, the antisense oligonucleotide may be modified to enhance in vivo stability relative to an unmodified oligonucleotide of the same sequence. The modification may be a (2'-O-(2-methoxyethyl)) modification. The oligonucleotide may have a phosphorothioate backbone throughout, the sugar moieties of nucleotides 1-4 and 18-21 may bear 2'-O-methoxyethyl modifications and the remaining nucleotides may be 2'-deoxynucleotides.

The antisense oligonucleotide may be a 5-10-5 gap-mer methoxyl ethyl modified (MOE) oligonucleotide corresponding to SEQ ID NO. 5 below. The antisense oligonucleotide may be from 10-25 bases in length, or from 15-23 bases in length, or from 18-22 bases in length, or 21 bases in length.

Exemplary sequences which can be employed as antisense oligonucleotides in the combination of the invention are disclosed in PCT Patent Publication WO 00/49937, US Patent Publication US-2002-0128220-A1 , and US Patent No. 6383808. Specific antisense oligonucleotide sequences are set forth in the present application as Seq. ID Nos: 2 to 19 and are represented in Table 1.

**Table 1**

| **SEQ ID No** | Description | SEQUENCE (5' to 3') |
|---|---|---|
| **2** | Antisense TRPM-2 oligonucleotide | GCACAGCAGG AGAATCTTCA T |
| **3** | Antisense TRPM-2 oligonucleotide | TGGAGTCTTT GCACGCCTCG G |
| **4** | Antisense oligonucleotide corresponding to the human TRPM-2 translation initiation site | CAGCAGCAG AGTCTTCATCA T |
| **5** | Antisense TRPM-2 oligonucleotide | ATTGTCTGAGACCGTCTGGTC |
| **6** | Antisense TRPM-2 oligonucleotide | CCTTCAGCTTTGTCTCTGATT |
| **7** | Antisense TRPM-2 oligonucleotide | AGCAGGGAGTCGATGCGGTCA |
| **8** | Antisense TRPM-2 oligonucleotide | ATCAAGCTGCGGACGATGCGG |
| **9** | Antisense TRPM-2 oligonucleotide | GCAGGCAGCCCGTGGAGTTGT |
| **10** | Antisense TRPM-2 oligonucleotide | TTCAGCTGCTCCAGCAAGGAG |
| **11** | Antisense TRPM-2 oligonucleotide | AATTTAGGGTTCTTCCTGGAG |
| **12** | Antisense TRPM-2 oligonucleotide | GCTGGGCGGAGTTGGGGGCCT |
| **13** | Antisense TRPM-2 oligonucleotide | GGTGTAGACG CCGCACG |
| **14** | Antisense TRPM-2 oligonucleotide | GCAGCGCAGC CCCTGG |
| **15** | Antisense TRPM-2 oligonucleotide | GCAGCAGCCG CAGCCCGGCT CC |
| **16** | Antisense TRPM-2 oligonucleotide | AGCCGCAGCC CGGCTCCT |
| **17** | Antisense TRPM-2 oligonucleotide | CAGCAGCCGC AGCCCGGCTC |
| **18** | Antisense TRPM-2 oligonucleotide | GCAGCAGCCG CAGCCCGGCT |
| **19** | Antisense TRPM-2 oligonucleotide | AGCAGCCGCAGCCCGGCTCC |
| **20** | 2 base TRPM-2 mismatch oligonucleotide used as a control | CAGCAGCAGAGTATTTATCAT |

A particularly preferred antisense oligonucleotide is a 21 mer oligonucleotide (CAGCAGCAGAGTCTTCATCAT; SEQ ID NO.: 4) targeted to the translation initiation codon and next 6 codons of the human clusterin sequence with a 2'-MOE modification. In one embodiment, this oligonucleotide has a phosphorothioate backbone throughout. The sugar moieties of nucleotides 1-4 and 18-21 (the "wings") bear 2'-O-methoxyethyl modifications and the remaining nucleotides (nucleotides 5-17; the "deoxy gap") are 2'-deoxynucleotides. Cytosines in the wings (i.e., nucleotides 1, 4 and 19) are 5-methylcytosines.

### RNAi oligonucleotides

Reduction in the amount of clusterin may also be achieved using RNA interference or "RNAi". RNAi is a term initially coined by Fire and co-workers to describe the observation that double-stranded RNA (dsRNA) can block gene expression[3]. Double stranded RNA, or dsRNA directs gene-specific, post-transcriptional silencing in many organisms, including vertebrates. RNAi involves mRNA degradation, but many of the biochemical mechanisms underlying this interference are unknown. The use of RNAi has been further described[3,4].

The initial agent for RNAi is a double stranded RNA molecule corresponding to a target nucleic acid. The dsRNA is then thought to be cleaved *in vivo* into short interfering RNAs (siRNAs) which are 21-23 nucleotides in length (19-21 bp duplexes, each with 2 nucleotide 3' overhangs). Alternatively, RNAi may be effected via directly introducing into the cell, or generating within the cell by introducing into the cell a suitable precursor (e.g. vector, etc.) of such an siRNA or siRNA-like molecule. An siRNA may then associate with other intracellular components to form an RNA-induced silencing complex (RISC).

RNA molecules used in embodiments of the present invention generally comprise an RNA portion and some additional portion, for example a deoxyribonucleotide portion. The total number of nucleotides in the RNA molecule is suitably less than 49 in order to be effective mediators of RNAi. In preferred RNA molecules, the number of nucleotides is 16 to 29, more preferably 18 to 23, and most preferably 21-23.

In certain embodiments of the invention, the siRNA or siRNA- like molecule is less than about 30 nucleotides in length. In a further embodiment, the siRNA or siRNA-like molecules are about 21 -23 nucleotides in length. In an embodiment, siRNA or siRNA-like molecules comprise and 19-21 bp duplex portion, each strand having a 2 nucleotide 3' overhang.

In certain embodiments of the invention, the siRNA or siRNA-like molecule is substantially identical to a clusterin-encoding nucleic acid or a fragment or variant (or a fragment of a variant) thereof. Such a variant is capable of encoding a protein having clusterin-like activity. In some embodiments, the sense strand of the siRNA or siRNA-like molecule being to the same target region as to the antisense species of SEQ ID NO. 4 or a fragment thereof (RNA having U in place of T residues of the DNA sequence). In other embodiments, the RNAi sequence consists of Seq. ID. No. 41 or 43. For example, United States published patent application 2004096882 discloses RNAi therapeutic probes targeting clusterin. In addition, reagents and kits for performing RNAi are available commercially from for example Ambion Inc. (Austin, TX, USA) and New England Biolabs Inc. (Beverly, MA, USA). Suitable sequences for use as RNAi in the present invention are set forth in the present application as Seq. ID Nos. 21 to 44 as shown in Table 2.

**Table 2**

| SEQ ID No. | Description | SEQUENCE |
|---|---|---|
| 21 | RNAi for human clusterin | GUAGAAGGGC GAGCUCUGGTT |
| 22 | RNAi for human clusterin | GAUGCUCAACACCUCCUCCT T |
| 23 | RNAi for human clusterin | GGAGGAGGUG UUGAGCAUCT T |
| 24 | RNAi for human clusterin | CUAAUUCAAU AAAACUGUCT T |
| 25 | RNAi for human clusterin | GACAGUUUUA UUGAAUUAGT T |
| 26 | RNAi for human clusterin | UAAUUCAACA AAACUGUTT |
| 27 | RNAi for human clusterin | ACAGUUUUGU UGAAUUATT |
| 28 | RNAi for human clusterin | AUGAUGAAGA CUCUGCUGCT T |
| 29 | RNAi for human clusterin | GCAGCAGAGU CUUCAUCAUT T |
| 30 | RNAi for human clusterin | UGAAUGAAGG GACUAACCUG TT |
| 31 | RNAi for human clusterin | CAGGUUAGUC CCUUCAUUCA TT |
| 32 | RNAi for human clusterin | CAGAAAUAGA CAAAGUGGGG TT |
| 33 | RNAi for human clusterin | CCCCACUUUG UCUAUUUCUG TT |
| 34 | RNAi for human clusterin | ACAGAGACUA AGGGACCAGA TT |
| 35 | RNAi for human clusterin | ACAGAGACUA AGGGACCAGA TT |
| 36 | RNAi for human clusterin | CCAGAGCUCG CCCUUCUACT T |
| 37 | RNAi for human clusterin | GUAGAAGGGC GAGCUCUGGT T |
| 38 | RNAi for human clusterin | GUCCCGCAUC GUCCGCAGCT T |
| 39 | RNAi for human clusterin | GCUGCGGACG AUGCGGGACT T |
| **40** | RNAi for human clusterin | CUAAUUCAAU AAAACUGUCT T |
| **41** | RNAi for human clusterin | GACAGUUUUA UUGAAUUAGT T |
| **42** | RNAi for human clusterin | AUGAUGAAGA CUCUGCUGC |
| **43** | RNAi for human clusterin | GCAGCAGAGU CUUCAUCAU |
| **44** | RNAi for human clusterin | CCAGAGCUCG CCCUUCUACT T |

### Cancers that can be treated

The combination of the present invention is useful in the treatment of a variety of cancers In which HER-2 Inhibition is significant. These cancers include breast cancer, osteosarcoma, lung cancer, pancreatic cancer, salivary gland cancer, colon cancer, prostate cancer, endometrial cancer, and bladder cancer.

A variety of reagents targeting the EGFr family have been tested for efficacy in the treatment of lung cancer. These reports are summarized in Tiseo (2004) [9]

Targeting of HER-2/neu expression has been shown to have therapeutic potential in controlling the development and progression of prostate cancer, DI Lorenzo (2004) [10]. Her-2/neu has also been shown to be a target in ovarian cancer, Xu (2003) [11]; salivary gland cancer, Scholl (2001)[12]: endometrial cancer Cianciulli (2003)[13] and Slomovitz (2004)[14]; pancreatic cancer, Baxevanis (2004) [15]; colon and colorectal cancer, Park (2004)[16], Half (2004) [17] and Nathanson (2003)[18]; and bladder cancer , Bellmunt (2003)[19].

### Methods

Administration of antisense oligonucleotides can be carried out using the various mechanisms known in the art, including naked administration and administration in pharmaceutically acceptable lipid carriers. For example, lipid carriers for antisense delivery are disclosed in US Patent Nos. 5,855,911 and 5,41 7,978. In general, the antisense is administered by intravenous, intraperitoneal, subcutaneous or oral routes, or direct local tumor injection.

The amount of antisense oligonucleotide administered is one effective to reduce the expression of clusterin in cancer cells, particularly and surprisingly when in combination with an agent that perturbs the HER-2 cell signaling pathway. It will be appreciated that this amount will vary both with the effectiveness of the antisense oligonucleotide employed, and with the nature of any carrier used. The determination of appropriate amounts for any given composition is within the skill in the art, through standard series of tests designed to assess appropriate therapeutic levels. In one embodiment, the antisense oligonucleotide is administered to a human patient in an amount of between 40-640 mg, or more particularly, from 300-640 mg. In another embodiment, the antisense oligonucleotide is administered according to the weight of the subject in need of the treatment. For example, the antisense oligonucleotide may be provided at a dosage of from 1 to 20 mg/kg of body weight.

The monoclonal trastuzumab is available as a powder in a vial containing 440 mg of drug. It must be mixed with a liquid before intravenous injection, often with an initial dose of 4 mg per kilogram of body weight followed by a weekly dose of 2 mg per kilogram of body weight. See, for example, Slamon, DJ et al., 2001 [5]

### Additional Therapeutic Agents

The combination according to the invention may be used for treating cancer with further administration of chemotherapy agents or other agents useful in breast cancer therapy and/or additional antisense oligonucleotides directed at different targets in combination with the therapeutic effective to reduce the amount of active clusterin. For example, antisense clusterin oligonucleotide may be used in combination with more conventional chemotherapy agents such as taxanes (paclitaxel or docetaxel), mitoxanthrone, doxorubicin, gemcitabine, cyclophosphamide, decarbazine, topoisomerase inhibitors), angiogenesis inhibitors, differentiation agents and signal transduction inhibitors.

The invention is further described in the following non-limiting examples.

### EXAMPLES

### Materials and Methods

### Tumour Cells

The BT474 human breast carcinoma cell line was cultured in DMEM supplemented with 10% fetal calf serum, glutamine, penicillin and streptomycin sulfate at 37°C under 5% CO₂-95% air. Cell culture reagents were purchased from Invitrogen (Milan, Italy).

### Reagents

Trastuzumab (Herceptin^{®}, purchased from Roche, Monza, Italy) was stored at 4°C and adjusted to the final concentration with culture medium.

Phosphorothioate oligonucleotides used in this study were purchased from La Jolla Pharmaceuticals Co. (La Jolla, CA, USA) or provided by OncoGenex Technologies Inc., Vancouver, Canada. The sequence of the clusterin ASO used corresponded to the human clusterin translation initiation site (5'-CAGCAGCAGAGTCTTCATCAT-3') (SEQ ID NO.:4). A 2-base clusterin mismatch oligonucleotide (5'-CAGCAGCAGAGT*A*TT*T*ATCAT-3') (SEQ ID NO.: 20) was used as control. Oligonucleotides were delivered into cells in form of complexes with the Lipofectin™ transfection reagent (Invitrogen). Cells were incubated with different concentrations of oligonucleotides and Lipofectin™ for 6 hours in OPTIMEM™ medium (Gibco). At the end of oligonucleotide treatment, the medium was replaced with fresh growth medium containing 2% of fetal calf serum and at different time points, cells were processed according to the various analyses to be performed.

### Proliferation assay

5 x 10⁵ cells were seeded in 60-mm culture dishes (Nunc™, Mascia Brunelli, Milano, Italy) and allowed to attach. Forty-eight hours after seeding, cells were the treated with clusterin ASO at 500nM for 6h. After oligonucleotide treatment, cells were exposed to a 25µM concentration of trastuzumab. At different time points during treatment, cells were harvested and assessed using a Cell Viability Analyzer™ (Coulter, Model Vi-Cell XR, Beckman, FL, USA).

### Western Blot

Western blot and detection were performed as previously reported, for example in [7]. Briefly, 40 µg of total proteins were loaded on denaturing SDS-PAGE. Immunodetection of clusterin and PARP proteins was performed by using, respectively, mouse anti-clusterin (1:1000, 41 D, Upstate Biotechnology, NY, USA) and rabbit anti-PARP (1:2000, VIC5, Boehringer Mannheim, Germany). To check the amount of proteins transferred to nitrocellulose membrane, HSP 72/73 was used as control and detected by an anti-human HSP 72/73 (1:1000, Calbiochem Cambridge, MA, USA). The relative amounts of the transferred proteins were quantified by scanning the autoradiographic films with a gel densitometer scanner (Bio-Rad, Milano, Italy) and normalized to the related HSP72/73 amounts.

### Cell cycle analysis

Measuring the percentage of cells in different phases of cell cycle was performed by flow cytometry (Becton-Dickinson, Heidelberg, Germany) as previously described Telford, W.G. , L.E. King , and P.J. Fraker (1992) Comparative evaluation of several DNA binding dyes in the detection of apoptosis-associated chromatin degradation by flow cytometry. Cytometry. 13: 137-143 [8]. Briefly, 2x10⁵ adherent cells were fixed and resuspended in a solution containing the dye propidium iodide (PI) at a concentration of 50 µg/ml. Percentages of cells in the different phases of the cell cycle were calculated using CELLQuest™ software (Becton Dickinson).

### Evaluation of apoptosis

Apoptosis was detected by flow cytometric analysis of sub-G1 peaks, and also analyzed by Annexin V-FITC *versus* PI assay (Vybrant™ Apoptosis Assay, V-13242, Molecular Probes, Eugene, OR, USA). Briefly, adherent cells were harvested, suspended in the annexin-binding buffer (1x10⁶cells/ml) and incubated with the annexin V-FITC and PI for 15 min, at room temperature in the dark, then immediately analyzed by flow-cytometry. The data are presented as bi-parametric dot plots showing the annexin V-FITC green fluorescence *versus* the PI red fluorescence.

### EXAMPLE 1

### Enhanced expression of clusterin after treatment with trastuzumab in BT474 HER-2 overexpressing breast cancer cells.

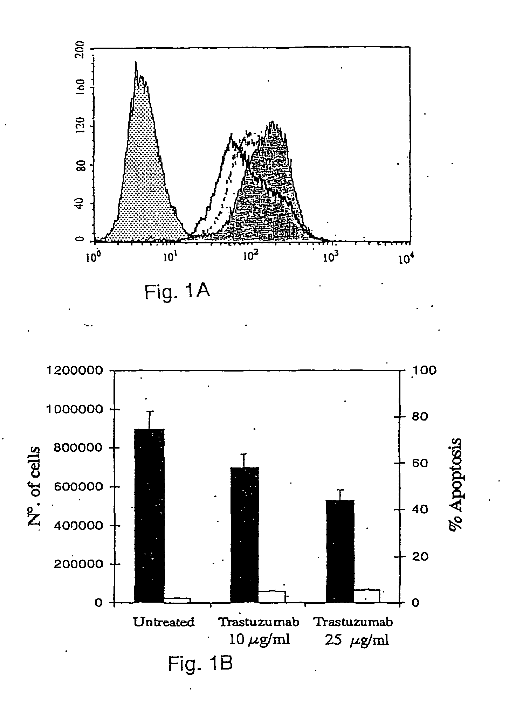

The human breast cancer cell line BT474, which overexpresses the HER-2 gene, was exposed to clinically relevant concentrations of trastuzumab. The treatment of BT474 cells with trastuzumab downregulated HER-2 protein expression in a dose-dependent manner. Analysis by flow cytometry revealed that the mean channel of fluorescence decreased from 173 to 112 and 72 in the BT474 cells treated with 10 and 25 µg/ml of trastuzumab, respectively. Figure 1A shows a cytofluorimetric analysis of HER-2 protein expression in BT474 cells untreated (gray area), and treated with 10 (thin line), 25 µg/ml (thick line) of trastuzumab for 48h, and negative control (dotted area).

Trastuzumab-mediated reduction of HER-2 protein expression was accompanied by inhibition of BT474 cell growth without affecting apoptosis (Fig 1 B). The simultaneous analysis of number of viable and apoptotic cells demonstrated that the treatment with trastuzumab significantly decreased cell growth in a dose- dependent effect, since the number of cells was reduced from about 1 x10⁶ to 8x10⁵ and 6x10⁵ in the BT474 cells treated with 10 and 25 µg/ml of trastuzumab, respectively. However, trastuzumab treatment even at the highest dose of 25 µg/ml induced little or no increase in the percentage of apoptotic cells (less than 10%).

it was next evaluated whether trastuzumab treatment was able to modulate clusterin/apolipoprotein J expression. Western blot analysis of clusterin protein in the lysates of BT474 cells exposed to trastuzumab (10-25 µg/ml) for up to 48h. The relative amount of the transferred clusterin protein was quantified and normalized relative to the corresponding HSP 72/73 protein amount. A representative of three independent experiments with similar results was evaluated. Both trastuzumab concentrations upregulated clusterin protein expression. At 25 µg/ml trastuzumab an increase of 3 fold of the 60 kD form and the appearance of the 40 kD form was observed.

### EXAMPLE 2

### Effect of combined treatment with clusterin ASO and trastuzumab on BT474 cell growth rate.

The effect of treatment with oligonucleotides on clusterin protein expression was evaluated. BT474 cells were transfected with 300 or 500 nM clusterin ASO (Seq. ID No. 4 with MOE modifications)(or the same concentrations of its mismatch oligonucleotide as control, as described *supra*)*,* and clusterin expression was analyzed by Western blot 48h after treatment. Treatment of BT474 with 100 or 500 nM clusterin ASO for 6h reduced clusterin protein expression by about 30 and 50% compared to mismatch control, respectively.

The analysis was performed 48h after the end of treatment. The relative amount of the transferred clusterin protein was quantified and normalized to the corresponding HSP72/73 protein amount.

In contrast, clusterin levels were not affected by the 2-base mismatch (MM) control oligonucleotide at any of the used concentrations.

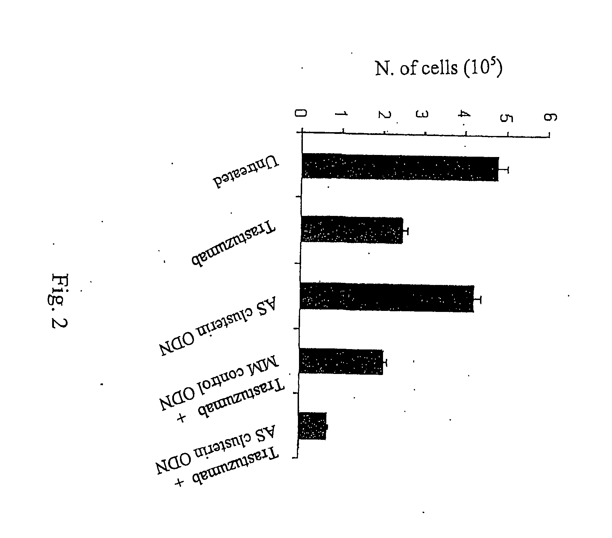

To determine whether treatment with clusterin ASO (Seq. ID No. 4, with MOE modification) enhances the cytotoxic effect of trastuzumab, BT474 cells were treated with 500 nM clusterin ASO or MM control oligonucleotide and then exposed to 25 µg/ml trastuzumab for 48h. Figure 2 shows the number of cells in the BT474 untreated and treated with trastuzumab alone and in combination with clusterin ASO or MM control oligonucleotide. Trastuzumab reduced the growth of BT474 by about 50%, while treatment with clusterin ASO did not show any effect on cell proliferation. The combination of trastuzumab with clusterin ASO, but not with the MM control oligonucleotide, significantly enhanced chemosensitivity of cells, with reduction of cell proliferation of up to 85%.

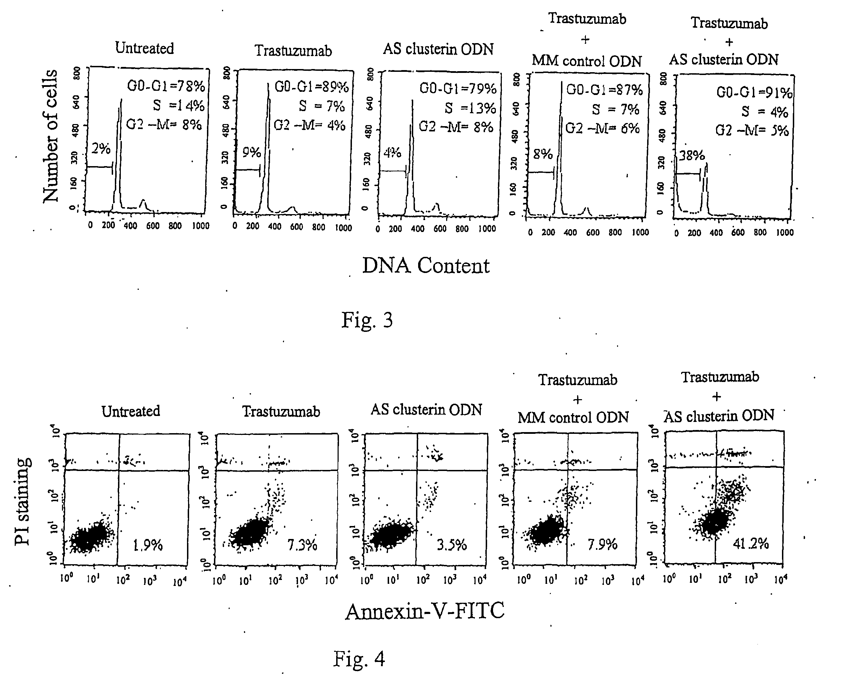

To study the mechanisms by which the treatment with clusterin ASO enhanced the chemosensitivity of cells to trastuzumab, cell cycle distribution was analyzed by flow cytometry. Figure 3 shows the histograms of DNA content in BT474 cells both untreated and treated with the single agents alone or in combination. The analysis was performed 48h after the end of treatments.

Analysis of cell percentages in the different phases of the cell cycle revealed that, while the treatment with clusterin ASO did not show any effect on cell cycle distribution, exposure of cells to trastuzumab induced a decrease of proliferative compartment (S-G₂/M) with the concomitant increase in the G₀/G₁ phase of the cell cycle. However, both treatments did not induce the appearance of populations with a sub-G1 DNA content. Cell cycle distribution of cells treated with trastuzumab plus MM control oligonucleotide was similar to that of trastuzumab alone. In contrast, a strong perturbation of the cell cycle was observed in the cells treated with trastuzumab and Clusterin ASO and a significant fraction of the cell population (about 40%) resided to the sub-G1 compartment.

### EXAMPLE 3

### Effect of combined treatment with clusterin ASO and trastuzumab on the induction of apoptosis.

Apoptosis was evaluated in BT474 cells exposed to the different treatments. Figure 4 shows the cytofluorimetric analysis of the annexin V *versus* PI staining performed in the BT474 untreated and treated with trastuzumab alone and in combination with Clusterin ASO or MM control oligonucleotide. Using the same treatment schedule described above, apoptosis (shown as the annexin V⁺/PI⁻ region of the dot plot panels) was observed only after combined treatment of Clusterin ASO plus trastuzumab. The percentage of annexin V⁺/PI⁻ cells was about 40% in the trastuzumab/clusterin combination and was less than 10% in all the other treatments.

### EXAMPLE 4

### Effect on Clusterin Protein Expression

The effect of combined clusterin ASO and trastuzumab on clusterin protein expression and PARP cleavage was also evaluated. Cells were treated with 500 nM of either clusterin ASO or MM control oligonucleotide for 6h, then exposed to 25 µg/ml of trastuzumab or control medium. Clusterin protein expression and poly (ADP-ribose) polymerase (PARP) cleavage were analyzed by Western blot. The relative amount of the transferred clusterin protein was quantified and normalized relative to the corresponding HSP 72/73 protein amount.

Expression of clusterin in the combination of trastuzumab plus MM control oligonucleotide is the same as that after treatment of trastuzumab alone, but the combination of trastuzumab with clusterin ASO blocked the trastuzumab-induced clusterin expression by specifically inhibiting its expression.

Analysis of the PARP cleavage demonstrated that the 116 KD intact form of PARP was observed in all samples examined, whereas the 85 KD PARP cleavage fragment was detected only after combined treatment with trastuzumab plus clusterin ASO.

While specific embodiments of the invention have been described and illustrated, such embodiments should be considered illustrative of the invention only and not as limiting the invention.

### References

1. Wong et al., Eur.J. Biochem. 221 (3), 917-925 (1994)
2. Sensibar et al., Cancer Research 55: 2431-2437 (1995)
3. Fire et al. (1998) Nature 391, 806-811.
4. Carthew et al. (2001) Current Opinions in Cell Biology 13, 244-248.
5. Slamon, D.J., Leyland-Jones, B., Shak, S., Fuchs, H., Paton, V., Bajamonde, A., Fleming, T., Elermann, W., Wolter, J., Pegram, M., Baselga, J., Norton, L. (2001) NEJM Vol 344(11):783-792
6. Elbashir et al. (2001) Nature 411, 494-498.
7. Miyake et al. (2000) Testosterone-repressed prostate message-2 is an antiapoptotic gene involved in progression to androgen independence in prostate cancer. Cancer Res. Jan 1; 60(1): 170-6.
8. Telford, W.G., L.E. King , and P.J. Fraker (1 992) Comparative evaluation of several DNA binding dyes in the detection of apoptosis-associated chromatin degradation by flow cytometry. Cytometry. 13: 137-143.
9. Tiseo M., Loprevite, M. and Ardizzoni, A. (2004) Epidermal Growth Factor receptor inhibitorsL a new prospective in the treatment of lung cancer. Curr. Med. Chem. Anti-Canc. Agents 4: 1 39-48.
10. Di Lorenzo et al. (2004) HER-2/neu receptor in prostate cancer development and progression to androgen independence. Tumori 90: 163-70.
11. Xu, X.F. and Xing, P.X. (2003) Discovery and validation of new molecular targets for ovarian cancer. Curr. Opin. Mol. Ther. 5: 625-630.
12. Scholl, S. Beuzeboc, P, and Pouillart, P. (2001) Targeting Her-2 in other tumor types. Ann Oncol. 12 Suppl 1: S81-7.
13. Cianciulli et al. (2003. Her-2/neu oncogene amplification and chromosome 1 7 aneusomy in endometrial carcinoma: correlation with oncoprotein expression and conventional pathological parameters. J. Exp. Clin. Cancer Res. 22: 265-71.
14. Slomovitz et al., (2004). Her-2/neu overexpression and amplification in uterine papillary serous carcinoma. J. Clin. Oncol. 22: 3126-32.
15. Baxevanis et al. (2004) Immunobiology of HER-2/neu oncoprotein and its potential in cancer immunotherapy. Cancer Immunol. Immunother. 53: 166-75.
16. Park et al. (2004) Clinical signficance of HER-2/neu expression in colon cancer. Korean J. Gastroenterol. 44: 147-52.
17. Half et al. (2004) HER-2 receptor expression, localization and activation in colorectal cancer cell lines and human tumors. Int. J. cancer 108: 540-8.
18. Nathanson et al. (2003) HER-2/neu expression and gene amplification in colon cancer. Int. J. cancer 105: 796-802.
19. Bellmunt, J, Hussain, M and Dinney, C.P. (2003). Novel approaches with targeted therapies in bladder cancer. Therapy of bladder cancer by blockade of the epidermal growth factor receptor family. Crit. Rev. Oncol. Hematol. 46 Suppl: S85-104.

## Claims

1. A combination for treating cancer in a mammalian subject, comprising
(i) an agent that is effective for treating cancer in the mammalian subject, and perturbs the HER-2 cell signalling pathway but increases the expression level of clusterin, the agent being a monoclonal antibody specific for HER-2, a small molecule inhibitor of HER-2, a peptide or peptide mimetic capable of interfering with HER-2, or an anti-HER-2 antisense oligonucleotide, and
(ii) an antisense oligonucleotide or an RNA interference inducing molecule, said oligonucleotide or RNA interference inducing molecule targeting clusterin mRNA and comprising nucleotides in a sequence that is complementary to clusterin mRNA, and further being effective to reduce the effective amount of clusterin in a cancer cell.

2. A combination according to claim 1, wherein the agent (i) that perturbs the HER-2 cell signalling pathway is a monoclonal antibody specific for HER-2.

3. A combination according to claim 2, wherein the antibody is trastuzumab.

4. A combination according to any one of claims 1 to 3, which comprises the antisense oligonucleotide which is an antisense clusterin oligonucleotide.

5. A combination according to claim 4, wherein the antisense clusterin oligonucleotide spans either the translation initiation site or the termination site of clusterin mRNA.

6. A combination according to claim 4 or 5, wherein the antisense clusterin oligonucleotide is modified to enhance *in vivo* stability relative to an unmodified oligonucleotide of the same sequence.

7. A combination according to claim 6, wherein the modification in the antisense clusterin oligonucleotide is a 2'-O-(2-methoxyethyl) modification.

8. A combination according to any one of claims 1 to 7, wherein the sequence of the antisense oligonucleotide is set forth in any one of Seq. ID Nos 2, 3, 6 to 11, or 13 to 19.

9. A combination according to any one of claims 1 to 7, wherein the sequence of the antisense oligonucleotide is set forth in Seq. ID No. 5 or Seq. ID No. 12.

10. A combination according to any one of claims 1 to 6, wherein the sequence of the antisense oligonucleotide is set forth in Seq. ID No. 4.

11. A combination according to claim 10, wherein the antisense oligonucleotide has a phosphorothioate backbone throughout, the sugar moieties of nucleotides 1-4 and 18-21 bear 2'-O-methoxyethyl modifications and the remaining nucleotides (nucleotides 5-17) are 2'-deoxynucleotides.

12. A combination according to any one of claims 1 to 3, wherein the RNA interference inducing molecule is an RNAi oligonucleotide comprising a sequence selected from Seq. ID Nos. 21 to 44.

13. Use of a combination according to any one of claims 1 to 12 for the treatment of cancer, such as osteosarcoma, lung cancer, pancreatic cancer, salivary gland cancer, colon cancer, prostate cancer, endometrial cancer or bladder cancer.

14. Use of a combination according to any of claims 1 to 12, for the treatment of breast cancer.

## Patentansprüche

1. Kombination zur Behandlung von Krebs in einem Säugetier umfassend
(i) einen Wirkstoff, der wirksam zur Behandlung von Krebs in dem Säugetier ist und den HER-2 Zellsignalweg stört, aber das Expressionslevel von Clusterin erhöht, der Wirkstoff, der ein monoklonaler Antikörper spezifisch für HER-2, ein niedermolekularer Inhibitor des HER-2, ein Peptid oder ein Peptid mimetisch fähig mit HER-2 zu interferieren oder ein anti-HER-2 Antisense-Oligonukleotid ist, und
(ii) ein Antisense-Oligonukleotid oder ein RNA Interferenz induzierendes Molekül, wobei das Oligonukleotid oder RNA Interferenz induzierende Molekül auf Clusterin mRNA abzielt und Nukleotide in einer Sequenz umfasst, die komplementär zur Clusterin mRNA ist und weiterhin wirksam ist die wirksame Menge an Clusterin in einer Krebszelle zu reduzieren.

2. Kombination nach Anspruch 1, wobei der Wirkstoff (i), der den HER-2 Zellsignalweg stört, ein monoklonaler Antikörper spezifisch für HER-2 ist.

3. Kombination nach Anspruch 2, wobei der Antikörper Trastuzumab ist.

4. Kombination nach einem der Ansprüche 1 bis 3, welche das Antisense-Oligonukleotid umfasst, welches ein Antisense-Clusterin-Oligonukleotid ist.

5. Kombination nach Anspruch 4, wobei das Antisense-Clusterin-Oligonukleotid entweder die Translationsinitiationsstelle oder die Terminationsstelle der Clusterin mRNA umspannt.

6. Kombination nach Anspruch 4 oder 5, wobei das Antisense-Clusterin-Oligonukleotid modifiziet ist, um die *in vivo* Stabilität relativ zu einem unmodifizierten Oligonukleotid von derselben Sequenz zu erhöhen.

7. Kombination nach Anspruch 6, wobei die Modifikation in dem Antisense-Clusterin-Oligonukleotid eine 2'-O-(2-methoxyethyl)-Modifikation ist.

8. Kombination nach einem der Ansprüche 1 bis 7, wobei die Sequenz von dem Antisense-Oligonukleotid in einer der SEQ. ID Nrn. 2, 3, 6 bis 11, oder 13 bis 19 gezeigt ist.

9. Kombination nach einem der Ansprüche 1 bis 7, wobei die Sequenz von dem Antisense-Oligonukleotid in der SEQ. ID Nr. 5 oder SEQ. ID Nr. 12 gezeigt ist.

10. Kombination nach einem der Ansprüche 1 bis 6, wobei die Sequenz von dem Antisense-Oligonukleotid in SEQ. ID Nr. 4 gezeigt ist.

11. Kombination nach Anspruch 10, wobei das Antisense-Oligonukleotid ein Phosphorothioat hat, die Zuckerhälften der Nukleotide 1-4 und 18-21 2'-O-methoxyethyl Modifikation tragen und die verbleibenden Nukleotide (Nukleotide 5-17) 2'-Deoxynukleotide sind.

12. Kombination nach einem der Ansprüche 1 bis 3, wobei die RNA Interferenz beinhaltendes Molekül ein RNAi Oligonukleotid ist umfassend eine Sequenz ausgewählt aus SEQ. ID Nrn. 21 bis 44.

13. Verwendung einer Kombination nach einem der Ansprüche 1 bis 12 für die Behandlung von Krebs wie Osteosarkomen, Lungenkrebs, Bauspeicheldrüsenkrebs, Speicheldrüsenkrebs, Darmkrebs, Prostatakrebs, Endometriumkrebs oder Blasenkrebs.

14. Verwendung von einer Kombination nach einem der Ansprüche 1 bis 12 für die Behandlung von Brustkrebs.

## Revendications

1. Combinaison pour le traitement du cancer chez un mammifère, comprenant
(i) un agent qui est efficace pour traiter le cancer chez le sujet mammifère, et perturbe la voie de signalisation de la cellule HER-2, mais augmente le niveau d'expression de la clustérine, l'agent étant un anticorps monoclonal spécifique de l'HER-2, un petit inhibiteur de la molécule d' HER-2, un peptide ou un peptide mimétique capable d'interférer avec HER-2, ou un oligonucléotide antisens anti-HER-2, et
(ii) un oligonucléotide antisens ou une molécule induisant une interférence par ARN, ledit oligonucléotide ou molécule induisant une interférence par ARN ciblant l'ARNm de la clustérine et comprenant des nucléotides dans une séquence qui est complémentaire de l'ARNm de la clustérine, et étant en outre efficace pour réduire la quantité effective de clustérine dans une cellule cancéreuse.

2. Combinaison selon la revendication 1, dans laquelle l'agent (i) qui perturbe la voie de signalisation de la cellule HER-2 est un anticorps monoclonal spécifique de l'HER-2.

3. Combinaison selon la revendication 2, dans laquelle l'anticorps est le trastuzumab.

4. Combinaison selon l'une quelconque des revendications 1 à 3, qui comprend l'oligonucléotide antisens qui est un oligonucléotide antisens de la clustérine.

5. Combinaison selon la revendication 4, dans laquelle l'oligonucléotide antisens de la clustérine couvre soit le site d'initiation de la traduction soit le site de terminaison de l'ARNm de la clustérine.

6. Combinaison selon la revendication 4 ou 5, dans laquelle l'oligonucléotide antisens de la clustérine est modifiée pour améliorer la stabilité *in vivo* par rapport à un oligonucléotide non modifié de la même séquence.

7. Combinaison selon la revendication 6, dans laquelle la modification de l'oligonucléotide antisens de la clustérine est une modification 2'-O-(2-méthoxyéthyle).

8. Combinaison selon l'une quelconque des revendications 1 à 7, dans laquelle la séquence de l'oligonucléotide antisens est présentée dans l'une quelconque des identifications de séquences n°2, 3, 6 à 11, ou 13 à 19.

9. Combinaison selon l'une quelconque des revendications 1 à 7, dans laquelle la séquence de l'oligonucléotide antisens est présentée dans l'identification de séquence n°5 ou dans l'identification de séquence n°12.

10. Combinaison selon l'une quelconque des revendications 1 à 6, dans laquelle la séquence de l'oligonucléotide antisens est présentée dans l'identification de séquence n°4.

11. Combinaison selon la revendication 10, dans laquelle l'oligonucléotide antisens a d'un bout à l'autre une structure phosphorothioate, les fragments de sucre de nucléotides 1-4 et 18-21 portent des modifications de 2'-O-méthoxyéthyle et les nucléotides restants (nucléotides 5-17) sont des 2'-désoxynucléotides.

12. Combinaison selon l'une quelconque des revendications 1 à 3, dans laquelle la molécule induisant une interférence par ARN est un oligonucléotide ARNi comprenant une séquence sélectionnée dans l'identification de séquence n°21 à 44.

13. Utilisation d'une combinaison selon l'une quelconque des revendications 1 à 12 pour le traitement du cancer, comme l'ostéosarcome, le cancer du poumon, le cancer du pancréas, le cancer des glandes salivaires, le cancer du côlon, cancer de la prostate, cancer de l'endomètre ou de cancer de la vessie.

14. Utilisation d'une combinaison selon l'une quelconque des revendications 1 à 12, pour le traitement du cancer du sein.


### SEQUENCE LISTING

<110> The university of British Columbia
<120> Treatment of cancer with a combination of an agent that perturbs the EGF signaling pathway and an oligonucleotide that reduces clusterin levels
<130> P1243EPPC
<140> 05810600.6
   <141> 2005-11-22
<150> US 60/522,948
   <151> 2004-11-23
<150> US 60/522,960
   <151> 2004-11-24
<160> 44
<170> PatentIn version 3.3
<210> 1
   <211> 2859
   <212> DNA
   <213> Homo sapiens
<400> 1
   ctttccgcgg cattctttgg gcgtgagtca tgcaggtttg cagccagccc caaagggggt    60
   gtgtgcgcga gcagagcgct ataaatacgg cgcctcccag tgcccacaac gcggcgtcgc   120
   caggaggagc gcgcgggcac agggtgccgc tgaccgaggc gtgcaaagac tccagaattg   180
   gaggcatgat gaagactctg ctgctgtttg tggggctgct gctgacctgg gagagtgggc   240
   aggtcctggg ggaccagacg gtctcagaca atgagctcca ggaaatgtcc aatcagggaa   300
   gtaagtacgt caataaggaa attcaaaatg ctgtcaacgg ggtgaaacag ataaagactc   360
   tcatagaaaa aacaaacgaa gagcgcaaga cactgctcag caacctagaa gaagccaaga   420
   agaagaaaga ggatgcccta aatgagacca gggaatcaga gacaaagctg aaggagctcc   480
   caggagtgtg caatgagacc atgatggccc tctgggaaga gtgtaagccc tgcctgaaac   540
   agacctgcat gaagttctac gcacgcgtct gcagaagtgg ctcaggcctg gttggccgcc   600
   agcttgagga gttcctgaac cagagctcgc ccttctactt ctggatgaat ggtgaccgca   660
   tcgactccct gctggagaac gaccggcagc agacgcacat gctggatgtc atgcaggacc   720
   acttcagccg cgcgtccagc atcatagacg agctcttcca ggacaggttc ttcacccggg   780
   agccccagga tacctaccac tacctgccct tcagcctgcc ccaccggagg cctcacttct   840
   tctttcccaa gtcccgcatc gtccgcagct tgatgccctt ctctccgtac gagcccctga   900
   acttccacgc catgttccag cccttccttg agatgataca cgaggctcag caggccatgg   960
   acatccactt ccatagcccg gccttccagc acccgccaac agaattcata cgagaaggcg  1020
   acgatgaccg gactgtgtgc cgggagatcc gccacaactc cacgggctgc ctgcggatga  1080
   aggaccagtg tgacaagtgc cgggagatct tgtctgtgga ctgttccacc aacaacccct  1140
   cccaggctaa gctgcggcgg gagctcgacg aatccctcca ggtcgctgag aggttgacca 1200
   ggaaatacaa cgagctgcta aagtcctacc agtggaagat gctcaacacc tcctccttgc 1260
   tggagcagct gaacgagcag tttaactggg tgtcccggct ggcaaacctc acgcaaggcg 1320
   aagaccagta ctatctgcgg gtcaccacgg tggcttccca cacttctgac tcggacgttc 1380
   cttccggtgt cactgaggtg gtcgtgaagc tctttgactc tgatcccatc actgtgacgg 1440
   tccctgtaga agtctccagg aagaacccta aatttatgga gaccgtggcg gagaaagcgc 1500
   tgcaggaata ccgcaaaaag caccgggagg agtgagatgt ggatgttgct tttgcaccta 1560
   cgggggcatc tgagtccagc tccccccaag atgagctgca gccccccaga gagagctctg 1620
   cacgtcacca agtaaccagg ccccagcctc caggccccca actccgccca gcctctcccc 1680
   gctctggatc ctgcactcta acactcgact ctgctgctca tgggaagaac agaattgctc 1740
   ctgcatgcaa ctaattcaat aaaactgtct tgtgagctga tcgcttggag ggtcctcttt 1800
   ttatgttgag ttgctgcttc ccggcatgcc ttcattttgc tatggggggc aggcaggggg 1860
   gatggaaaat aagtagaaac aaaaaagcag tggctaagat ggtataggga ctgtcatacc 1920
   agtgaagaat aaaagggtga agaataaaag ggatatgatg acaaggttga tccacttcaa 1980
   gaattgcttg ctttcaggaa gagagatgtg tttcaacaag ccaactaaaa tatattgctg 2040
   caaatggaag cttttctgtt ctattataaa actgtcgatg tattctgacc aaggtgcgac 2100
   aatctcctaa aggaatacac tgaaagttaa ggagaagaat cagtaagtgt aaggtgtact 2160
   tggtattata atgcataatt gatgttttcg ttatgaaaac atttggtgcc cagaagtcca 2220
   aattatcagt tttatttgta agagctattg cttttgcagc ggttttattt gtaaaagctg 2280
   ttgatttcga gttgtaagag ctcagcatcc caggggcatc ttcttgactg tggcatttcc 2340
   tgtccaccgc cggtttatat gatcttcata cctttccctg gaccacaggc gtttctcggc 2400
   ttttagtctg aaccatagct gggctgcagt accctacgct gccagcaggt ggccatgact 2460
   acccgtggta ccaatctcag tcttaaagct caggcttttc gttcattaac attctctgat 2520
   agaattctgg tcatcagatg tactgcaatg gaacaaaact catctggctg catcccaggt 2580
   gtgtagcaaa gtccacatgt aaatttatag cttagaatat tcttaagtca ctgtcccttg 2640
   tctctctttg aagttataaa caacaaactt aaagcttagc ttatgtccaa ggtaagtatt 2700
   ttagcatggc tgtcaaggaa attcagagta aagtcagtgt gattcactta atgatataca 2760
   ttaattagaa ttatggggtc agaggtattt gcttaagtga tcataattgt aaagtatatg 2820
   tcacattgtc acattaatgt caaaaaaaaa aaaaaaaaa 2859
<210> 2
   <211> 21
   <212> DNA
   <213> Homo sapiens
<400> 2
   gcacagcagg agaatcttca t 21
<210> 3
   <211> 21
   <212> DNA
   <213> Homo sapiens
<400> 3
   tggagtcttt gcacgcctcg g 21
<210> 4
   <211> 21
   <212> DNA
   <213> Homo sapiens
<400> 4
   cagcagcaga gtcttcatca t 21
<210> 5
   <211> 21
   <212> DNA
   <213> Homo sapiens
<400> 5
   attgtctgag accgtctggt c 21
<210> 6
   <211> 21
   <212> DNA
   <213> Homo sapiens
<400> 6
   ccttcagctt tgtctctgat t 21
<210> 7
   <211> 21
   <212> DNA
   <213> Homo sapiens
<400> 7
   agcagggagt cgatgcggtc a 21
<210> 8
   <211> 21
   <212> DNA
   <213> Homo sapiens
<400> 8
   atcaagctgc ggacgatgcg g 21
<210> 9
   <211> 21
   <212> DNA
   <213> Homo sapiens
<400> 9
   gcaggcagcc cgtggagttg t 21
<210> 10
   <211> 21
   <212> DNA
   <213> Homo sapiens
<400> 10
   ttcagctgct ccagcaagga g 21
<210> 11
   <211> 21
   <212> DNA
   <213> Homo sapiens
<400> 11
   aatttagggt tcttcctgga g 21
<210> 12
   <211> 21
   <212> DNA
   <213> Homo sapiens
<400> 12
   gctgggcgga gttgggggcc t 21
<210> 13
   <211> 17
   <212> DNA
   <213> Homo sapiens
<400> 13
   ggtgtagacg ccgcacg . 17
<210> 14
   <211> 16
   <212> DNA
   <213> Homo sapiens
<400> 14
   gcagcgcagc ccctgg 16
<210> 15
   <211> 22
   <212> DNA
   <213> Homo sapiens
<400> 15
   gcagcagccg cagcccggct cc 22
<210> 16
   <211> 18
   <212> DNA
   <213> Homo sapiens
<400> 16
   agccgcagcc cggctcct 18
<210> 17
   <211> 20
   <212> DNA
   <213> Homo sapiens
<400> 17
   cagcagccgc agcccggctc 20
<210> 18
   <211> 20
   <212> DNA
   <213> Homo sapiens
<400> 18
   gcagcagccg cagcccggct 20
<210> 19
   <211> 20
   <212> DNA
   <213> Homo sapiens
<400> 19
   agcagccgca gcccggctcc 20
<210> 20
   <211> 21
   <212> DNA
   <213> Homo sapiens
<400> 20
   cagcagcaga gtatttatca t 21
<210> 21
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 21
   guagaagggc gagcucuggt t 21
<210> 22
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 22
   gaugcucaac accuccucct t 21
<210> 23
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 23
   ggaggaggug uugagcauct t 21
<210> 24
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 24
   cuaauucaau aaaacuguct t 21
<210> 25
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 25
   gacaguuuua uugaauuagt t 21
<210> 26
   <211> 19
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 26
   uaauucaaca aaacugutt 19
<210> 27
   <211> 19
   <212> DNA
   <213> Homo sapiens
<220>
   <221> misc_feature
   <222> (1)..(17)
   <223> ribonucleotides
<220>
   <221> misc_feature
   <222> (18)..(19)
   <223> deoxynucleotides
<400> 27
   acaguuuugu ugaauuatt 19
<210> 28
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 28
   augaugaaga cucugcugct t 21
<210> 29
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 29
   gcagcagagu cuucaucaut t 21
<210> 30
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 30
   ugaaugaagg gacuaaccug tt 22
<210> 31
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 31
   cagguuaguc ccuucauuca tt 22
<210> 32
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 32
   cagaaauaga caaagugggg tt 22
<210> 33
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 33
   ccccacuuug ucuauuucug tt 22
<210> 34
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 34
   acagagacua agggaccaga tt 22
<210> 35
   <211> 22
   <212> DNA
   <213> Homo sapiens
<220>
   <221> misc_feature
   <222> (1)..(20)
   <223> ribonucleotides
<220>
   <221> misc_feature
   <222> (21)..(22)
   <223> deoxynucleotides
<400> 35
   acagagacua agggaccaga tt 22
<210> 36
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 36
   ccagagcucg cccuucuact t 21
<210> 37
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 37
   guagaagggc gagcucuggt t 21
<210> 38
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 38
   gucccgcauc guccgcagct t 21
<210> 39
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 39
   gcugcggacg augcgggact t 21
<210> 40
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 40
   cuaauucaau aaaacuguct t 21
<210> 41
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 41
   gacaguuuua uugaauuagt t 21
<210> 42
   <211> 19
   <212> DNA
   <213> Homo sapiens
<220>
   <221> misc_feature
   <222> (1)..(17)
   <223> ribonucleotides
<220>
   <221> misc_feature
   <222> (18)..(19)
   <223> ribonucleotides
<400> 42
   augaugaaga cucugcugc 19
<210> 43
   <211> 19
   <212> DNA
   <213> Homo sapiens
<220>
   <221> misc_feature
   <222> (1)..(17)
   <223> ribonucleotides
<220>
   <221> misc_feature
   <222> (18)..(19)
   <223> ribonucleotides
<400> 43
   gcagcagagu cuucaucau 19
<210> 44
   <211> 21
   <212> DNA
   <213> Homo sapiens
<220>
   <221> misc_feature
   <222> (1)..(19)
   <223> ribonucleotides
<220>
   <221> misc_feature
   <222> (20)..(21)
   <223> deoxynucleotides
<400> 44
   ccagagcucg cccuucuact t 21